# EUROPEAN PATENT SPECIFICATION

(11) **EP 4 215 613 B1**
(45) Date of publication and mention of the grant of the patent: **04.03.2026**
(21) Application number: 22152930.8
(22) Date of filing: 24.01.2022
(51) Int. Cl.: C12N 15/10, B01D 15/16, B01D 15/38

(54) **METHOD OF SEPARATING SINGLE STRANDED RNA MOLECULES FROM OTHER NUCLEIC ACID SPECIES, USE OF SOLID PHASE THEREFOR, AND METHOD OF SEPARATING DOUBLE STRANDED RNA MOLECULES BY SIZE**
VERFAHREN ZUR TRENNUNG VON EINZELSTRÄNGIGEN RNA-MOLEKÜLEN VON ANDEREN NUKLEINSÄURESPEZIES, VERWENDUNG EINER FESTPHASE DAFÜR UND VERFAHREN ZUR TRENNUNG VON DOPPELSTRÄNGIGEN RNA-MOLEKÜLEN NACH GRÖSSE
PROCÉDÉ DE SÉPARATION DE MOLÉCULES D'ARN SIMPLE BRIN À PARTIR D'AUTRES ESPÈCES D'ACIDE NUCLÉIQUE, UTILISATION D'UNE PHASE SOLIDE À CET EFFET ET PROCÉDÉ DE SÉPARATION DE MOLÉCULES D'ARN DOUBLE BRIN PAR TAILLE

(43) Date of publication of application: 26.07.2023
(73) Proprietor: Sartorius BIA Separations d.o.o., 5270 Ajdovscina (SI)
(72) Inventor: Cernigoj, Urh, Ajdovscina (SI); Blaz, Bakalar, Ajdovscina (SI); Miklavcic, Rok, Sempas (SI); Gagnon, Peter Stanley, Las Vegas (US); Dolenc, Darko, Vrhnika (SI)
(74) Representative: Novagraaf International SA

(56) References cited:
- US-A1- 2001 018 513
- US-A1- 2017 298 413
- SOUSA A ET AL: "Histamine monolith versatility to purify supercoiled plasmid deoxyribonucleic acid from Escherichia coli lysate", JOURNAL OF CHROMATOGRAPHY A, ELSEVIER, AMSTERDAM, NL, vol. 1355, 6 June 2014 (2014-06-06), pages 125 - 133, XP029034352, ISSN: 0021-9673, DOI: 10.1016/J.CHROMA.2014.06.003

## Description

The present invention relates to a method of separating single-stranded RNA (ssRNA) molecules from other nucleic acid species, especially double-stranded RNA (dsRNA) species, and to the use of a solid phase for separating single-stranded RNA from other nucleic acid species. The present invention further relates to a method of separating double stranded RNA molecules by size.

There is a growing need for a greater variety of separation methods in RNA and DNA related processes for biopharma drug substance production. As an example, in messenger RNA (mRNA) production, the rapid development of mRNA-based vaccines and the large scale production thereof has become increasingly important as shown by the recent COVID-19 outbreak. Synthesis of mRNA for gene therapy and vaccination applications via in vitro transcription (IVT) yields preparations that contain undesired subpopulations of different nucleic acid species including double-stranded (ds) RNA in addition to the desired single-stranded (ss) RNA. dsRNA triggers an unwanted immune response when injected into a subject, making its removal a particular object of purification.

mRNA preparations also contain residual dsDNA from the plasmid template (pDNA) used to synthesize the mRNA. dsDNA levels can be lowered through nuclease enzyme treatment after transcription, but dsDNA fragments can still be present in the preparations. mRNA preparations further contain proteins that must be removed to lower the chance of patients developing non-specific immune response to them. These proteins include enzymes such as RNA polymerase, pyrophosphatase and nucleases used to degrade the dsDNA template after transcription.

Standard laboratory purification of RNA employs DNase enzymatic treatment to degrade DNA, followed by LiCl precipitation to purify and concentrate RNA. This approach offers a cheap and fast purification of RNA, however it is chemically harsh and difficult to scale up and requires subsequent removal of the nuclease.

Methods for reducing the content of dsRNA from mRNA preparations are known. For example, levels of dsRNA contamination can be reduced by affinity-adsorption chromatography on cellulose based chromatography media. Such methods are simple and effective on laboratory scale, but with a very low capacity, thus burdening the scaling up. Chromatography on the other hand offers a selective and easily scalable approach for manufacturing of highly pure preparations. Different approaches have been developed for RNA purification as described below.

Levels of dsRNA and dsDNA in mRNA preparations may be reduced by affinity chromatography using an oligo-deoxythymidine (dT) functionalized solid phase. This approach utilizes specific oligo-dT ligands, which hybridize with poly-adenine (A) regions of nucleic acids, thus enabling separation of mRNA due to its poly-A tail. Poly-A regions are, however, also present in template dsDNA and in generated dsRNA populations which all at least partially share the sequence of the target mRNA and in some conditions do not separate completely from the mRNA. Additionally, oligo-dT ligand production could become a global scale bottleneck due to an increasing need for mRNA therapeutics. Another downside of the approach is its uselessness for separation of ssRNAs lacking poly-A tails, which are not a negligible subgroup.

The primary and secondary amine-based chromatography enables separation of mRNA from oligonucleotides, enzymes and DNA at room temperature. Such methods employ a pH gradient for eluting ssRNA in the range of pH above 9.5, which is a drawback as mRNA is prone to hydrolysis at elevated concentrations of OH⁻ ions.

Additionally, a pyrophosphate anion gradient can be employed in primary and secondary amine based chromatography allowing elution of ssRNA in neutral pH at ambient temperature. The pyrophosphate is not widely recognized as a common anion in chromatography, which represents a burden at least for preparative applications. In addition, it is not hydrolytically stable in aqueous solutions, thus decreasing its potential for a wide usage.

Hydrophobic interaction chromatography (HIC) uses high concentrations of salts in mobile phases, whereby mRNA precipitation could occur in the solution or on the column. High salt buffers also increase the pressure on the chromatographic system, limiting the flow rate to lower values. Reversed phase chromatography on the other hand employs organic solvents, that are better avoided for GMP production, and high temperatures for successful binding and elution of ribonucleic acids, a big obstacle in biopharmaceutical manufacturing for human use.

Standard ion exchangers, specifically quaternary and tertiary amine ion exchangers, have been used for dsRNA separation in ascending NaCl gradient, but low recoveries were found for ssRNA. To successfully elute ssRNA above 1000 bases from standard ion exchangers, high temperature (e.g. 65 °C) and high concentration of toxic organic denaturants (e.g. acetonitrile) have to be used.

The state of the art further teaches that solid phase media derivatized with nitrogen-containing heterocycles, for example histamine-modified chromatographic columns, have been successfully applied for dsDNA (pDNA) separation from fragmented RNA, for example such as is present in E. *coli* lysate. In one example, HIC principle alone or in a combination with descending pH gradient was used with histamine- and histidinemodified chromatographic columns. The same principle is not applicable with ssRNA, especially above 1000 bases, because mRNA precipitation occurs in the buffers containing a concentration range of ammonium sulfate applied. In other applications with histamine-modified chromatographic columns, ion-exchanging conditions in ascending salt gradient were applied for the pDNA separation. The same principle is not applicable with mRNA, because low mRNA recovery from the column is achieved, similar to the separation efficiency on standard anion exchangers.

Sousa A. et al. (Journal of Chromatography A, vol. 1335, pages 125-133) describe histamine monolith versatility to purify supercoiled plasmid deoxyribonucleic acid from Escherichia coli lysate. US 2001/018513 A1 describes the isolation of nucleic acids. US 2017/298413 A1 describes methods for the isolation of biomolecules and uses thereof.

Surprisingly and in contrast to the teaching of the prior art, the inventors have found that a solid phase comprising a nitrogen-containing heterocycle on a surface as a multimodal ligand could solve the aforementioned issues and were able to show that such a solid phase behaves in contrast with the aforementioned expectations. In particular, the inventors of the present invention discovered that solid phase media derivatized with a multimodal ligand comprising a nitrogen-containing heterocycle enabled the development of a method that was able to overcome several practical limitations associated with the existing methods for separating ssRNA from other nucleic acids.

Based on the above, the object of the invention is to provide an inexpensive method of separating ssRNA from other nucleic acids and biomolecules which achieves a high separation resolution as well as a high recovery rate of the ssRNA at mild working conditions, i.e. ambient temperature of 15 to 30 °C, close to neutral pH and without chemicals that should be avoided for GMP production. Such chemicals are, for example, summarized in guidelines of the European Medicines Agency (EMA), such as EMA/CHMP/ICH/82260/2006 - ICH guideline Q3C (R8) on impurities: guideline for residual solvents.

According to the present invention, the above object is achieved by providing a method of separating single-stranded RNA from other nucleic acid species, comprising the steps of:
a) providing a solid phase comprising a multimodal ligand having a pKa value of 4.0 to 8.5, the multimodal ligand comprising at least one nitrogen-containing heterocycle;
b) loading a sample to the solid phase, the sample containing the target single-stranded RNA molecules to be separated; and
c) separating and eluting the target single-stranded RNA molecules by applying an ascending pH gradient nearing or crossing the pKa value of the multimodal ligand.

The surprising advantages of the invention include that ssRNA can be eluted from the solid phase comprising a multimodal ligand comprising a nitrogen-containing heterocycle with: 1) a high product recovery at room temperature, 2) in the proximity of the neutral pH, 3) without harmful additives. The combination of all three benefits is not known from prior art.

The separation method of the present invention employs a solid phase comprising a multimodal ligand. The multimodal ligand comprises at least one nitrogen-containing heterocycle as described above. In a preferred embodiment the solid phase is a support matrix having the multimodal ligands bound thereto. The type of solid phase is not particularly limited as long as it is suitable to be used for liquid chromatography and can comprise the multimodal ligands.

Preferably, the solid phase has on its surface chemical functional groups, to which the multimodal ligands can bind or be bound. These groups can already be present on the surface of the solid phase or can be introduced in a suitable manner. According to the invention, therefore, one can either use a chromatographic solid phase that originally comprises functional groups (such as glycidyl-containing polymethacrylates), or a chromatographic support into which suitable functional groups can be introduced by means of a surface modification known to the person skilled in the art. In this connection, examples of known surface modifications include substitution and addition reactions, reaction with functional epoxides, activated acids or active esters, activation by means of plasma treatment, e-beam (electron beam) treatment, gamma irradiation, coating, hydrolysis, aminolysis, oxidation, reduction, reaction with functional carbenes and/or nitrenes, etc.

According to an embodiment of the present invention, the solid phase comprises at least one material selected from the group composed of natural or synthetic fibers, (polymer) membranes, porous (polymer) particles, monolithic solid supports, polymer gels, films, nonwovens and wovens. In a preferred embodiment, the solid phase is a monolithic solid support.

Examples of natural or synthetic fibers that can be used as a material for the solid phase include electrospun cellulose fibers (such as "Fibro" from Cytiva, polyester fibers (such as "Winged Fibers" from the firm Allasso Industries, composed of polyethylene terephthalate (PET) or "4DG^{™} Fibers" from the firm Fiber Innovation Technology, composed of polyethylene terephthalate) and fibers comprising cellulose derivatives, Nylon, polyethylene (PE), polyamide (PA), sulfone (PES), polyvinylidene difluoride (PVDF), polytetrafluoroethylene (PTFE), polypropylene (PP) and polysulfone as a structuring component, wherein the materials can be used individually or in corresponding combinations.

Examples of (polymer) membranes that can be used as a material for the solid phase include membranes comprising cellulose, cellulose derivatives, Nylon, polyester, polyethylene (PE), polyamide (PA), sulfone (PES), polyvinylidene difluoride (PVDF), polytetrafluoroethylene (PTFE), polypropylene (PP) and polysulfone as a structuring component, wherein the materials can be used individually or in corresponding combinations. Preferably, membranes based on cellulose and cellulose derivatives, in particular cellulose hydrate membranes, or polyethylene membranes are used.

Examples of polymer gels that can be used as a material for the solid phase include agarose, dextran, cellulose, polymethacrylates, polyvinyl ethers, polyacrylamides, polystyrene-divinylbenzene copolymers, silica dextran, agarose acrylamides and dextran acrylamides.

Examples of films and wovens that can be used as a material for the solid phase include films and wovens composed of the above-mentioned polymer materials that can be used for the (polymer) membranes. Examples of nonwovens that can be used as a material for the solid phase include polyester/polypropylene/polyamide nonwovens (such as "Pluratexx 2317 S" from the firm Freudenberg) and the above polymer materials that can be used for the (polymer) membranes.

Examples of monolithic solid supports include molded monoliths, synthesized, for example, from a single polymer mixture composed of poly(glycidyl methacrylate -co-ethylene dimethacrylate). A particular example are CIM monolithic chromatographic columns from BIA Separations. Another example are so-called hydrogels, which represent monoliths synthesized first as a macro-skeleton with a secondary ligandbearing polymer phase synthesized on top of it. Solid support materials may be provided in a housing to facilitate performance of chromatography.

The above described materials may intrinsically comprise a multimodal ligand or may be functionalized with a multimodal ligand. According to the present invention, multimodal ligands comprising at least one nitrogen-containing heterocycle are bound to the surface of the material for the solid phase either directly or via polymeric spacer elements so as to form the solid phase.

In the solid phase used in the method of the present invention, the multimodal ligands are preferably bound via polymeric spacer elements between the surface of the material for the solid phase and the multimodal ligand to form the solid phase. The binding between the surface of the material for the solid phase and the spacer elements preferably takes place or has taken place via functional groups originally present or produced by surface modification of the solid phase. By means of the polymeric spacer elements, which may serve as binding units between the surface of the material for the solid phase and the multimodal ligands in the solid phase, it is advantageously possible to obtain high ligand densities, which allow high binding capacity for the target substance.

Within the meaning of the present invention, the term "multimodal" is understood to mean that the ligands comprise two or more different functionalities, which thus undergo interactions with the target single stranded RNA molecules based on different chemical and/or physical mechanisms, so that the latter are bound to the solid phase. Examples of these interactions include hydrophobic interactions, electrostatic interactions, ionic interactions, hydrogen bonding and π-π-interactions.

According to the invention, the multimodal ligand has a pKa value of 4.0 to 8.5 and comprises at least one nitrogen-containing heterocycle. According to the present invention, the multimodal ligand having a pKa value of 4.0 to 8.5 means that the at least one nitrogen-containing heterocycle has at least one nitrogen atom with a pKa value of the conjugate acid in the range of 4.0 to 8.5. The pKa value of the multimodal ligand can, for example, be determined by potentiometric titration at ambient temperature.

Nitrogen-containing heterocycle-comprising multimodal ligands are capable of multimodal interactions that can fine-tune the separation of chemically similar RNA/DNA species due to 3 crucial reasons:
Firstly, they can be neutral or positively charged at a specific pH. Their pKa values depend on the number and position of nitrogen atoms inside the ring as well as the ring size. According to the present invention, the ring structure is such that the pKa of the multimodal ligand when bound to a solid phase, is between 4.0 and 8.5. In a preferred embodiment, the pKa value is between 5.0 and 8.0. The charge of the multimodal ligand changes with pH. When the pH is below the pKa, the multimodal ligand is positively charged ("binding mode" for nucleic acids). The charge practically decreases to zero when the pH rises 2 units above the pKa value of the multimodal ligand ("elution mode"). The positive charge helps the nitrogen-containing heterocycles comprised in the multimodal ligands on the solid phase to interact with the backbone of nucleic acids, specifically with the negatively charged phosphate backbone. The charge switch point being close to or slightly below neutral pH is a key feature for optimal elution conditions, as single stranded RNA molecules are unstable after prolonged exposure to high pH conditions. Elution of bound nucleic acids is achieved by an ascending pH gradient nearing or crossing the pKa value of the multimodal ligand while maintaining constant conductivity of the running buffers. In another embodiment, the ascending pH gradient is performed in combination with an ascending or descending salt gradient. Accordingly, elution in neutral pH range is possible.

According to the present invention, a pH gradient nearing the pKa value of the multimodal ligand is a pH gradient that starts at a pH value below the pKa value of the multimodal ligand and finishes at a pH value that is close to the pKa value of the multimodal ligand but does not reach the pKa value of the multimodal ligand. Similarly, a pH gradient crossing the pKa value of the multimodal ligand is a pH gradient that starts at a pH value below the pKa value of the multimodal ligand and finishes at a pH value that is equal to or higher than the pKa value of the multimodal ligand.

Secondly, nitrogen-containing heterocycles can form hydrogen bonds with nucleic acid residues. This mode of interaction fine-tunes the strength of binding and helps separating otherwise chemically very similar species like mRNA, linear DNA (linDNA), dsRNA, pDNA etc, due to each species having a different profile and potential for hydrogen bonding. The relative strength of hydrogen bonding is higher at lower pH, thus enabling ascending pH gradient as a preferential elution mechanism. This type of interactions, however, is not possible with quaternary ammonium groups.

Thirdly, nitrogen-containing heterocycles may have aromatic properties and thus exhibit hydrophobic interactions with aromatic bases of nucleic acids in salt exclusion conditions. The potential for salt-exclusion based interactions is heavily influenced by "strand flexibility" and is higher for ssRNA (mRNA) as compared to double stranded counterparts, dsRNA and linDNA, which are much more rigid in structure and limit the hydrophobic interaction potential to the grooves.

The relative strength of salt-exclusion based interactions is higher at higher pH due to the deprotonation of the nitrogen-containing heterocycles. A pH gradient therefore enables nucleic acid species separation based on their relative hydrophobicity.

Accordingly, the method of the present invention is capable of selectively separating nucleic acid molecules at least based on their charge and their capability of forming hydrogen bonds. The method of the present invention is further capable of separating nucleic acids by hydrophobic interactions.

In a preferred embodiment, the nitrogen-containing heterocycle is an aromatic nitrogen-containing heterocycle. In this case the multimodal ligand is additionally sensitive to the aromatic properties of the nucleic acid molecules.

In particular, an important aspect of aromatic nitrogen-containing heterocycles is the ability to delocalize charge, which alters their interaction properties compared to other amine ligands, including primary-, secondary-, tertiary- and quaternary ammonium ligands.

According to the present invention, an ascending pH gradient nearing or crossing the pKa value of the multimodal ligand is used. Conventionally, a pH gradient as used in biochromatography of proteins is applied within a pH range, where the stationary phase is charged and stable, without crossing the pKa of the multimodal ligand. With anion exchangers it is therefore advisable to start with a high pH, where electronegative proteins bind to the positively charged stationary phase, and then decrease the pH until the proteins elute due to electrostatic repulsion when becoming more electropositive. The initial and final pH values are both below the multimodal ligand pKa. The separation mechanism follows the charge change of the analyte, according to the respective isoelectric points (pl) of the molecules. This principle, however, does not work with nucleic acids, because their pl is very low (2-3) and similar for RNA and DNA molecules.

According to the present invention, the multimodal ligands have a pKa value of 4.0 to 8.5. When applying an ascending pH gradient nearing or crossing the pKa of the multimodal ligand, the multimodal ligand loses its charge, thereby decreasing the interaction between the multimodal ligand and nucleic acid. This mechanism is not possible with strong anion exchangers, because they do not lose charge. On the other hand, with weak anion exchangers such as simple amine ligands (e.g. diethylaminoethyl, diethylaminopropyl, etc.), which have a pKa above 8.5, the nucleic acid desorption occurs at high pH (above 10). This is not desirable due to lower chemical stability of single stranded RNA after prolonged exposure to such alkaline pH. Since, according to the present invention, the multimodal ligand has a pKa value of 4.0 to 8.5 and thus transitions from a positive to neutral charge at neutral or slightly acidic conditions. This enables the elution of nucleic acids from slightly acidic/neutral to slightly alkaline pH (below pH 9), where nucleic acids and especially mRNA are relatively stable.

Here, it is important to stress that while charge transition will elute nucleic acids, this alone is not sufficient to achieve substantial separation between different groups of DNA or RNA molecules. This is why additional multimodal interactions are necessary to fine-tune and improve the separation of these very similar chemical species.

Accordingly, the present invention provides nucleic acid separation based on a combination of specific multimodal ligands and an ascending pH gradient. The usage of an ascending gradient over a pH range, where ligand charge loss happens, allows for distinct "bind" and "elute" modes of operation, together with influencing hydrogen bonding, salt-exclusion based interactions and other above described interactions, which determine the fine separation of similar species (RNA and DNA).

According to the present invention, ssRNA separation from other nucleic acids is at least partially based on hydrogen bonding between lone electron pairs or acidic hydrogen of the conjugated acid of the nitrogen-containing heterocycle comprised in the multimodal ligand with different functional groups on nucleic acids. These interactions are stronger at lower pH, therefore an ascending gradient enables desorption of the bound molecules. dsRNA/DNA molecules have their bases paired and consequently hidden in the core of a double helix. Additionally, double stranded species are much more rigid and cannot conform to the surface of the solid phase having the multimodal ligands bound thereto as easily and have a weaker interaction with the solid phase as a consequence. Base-paired bases are also not fully available for hydrogen-bonding interaction with the multimodal ligand on a solid phase, hence dsRNA/DNA interact to a greater extent with the solid phase through the electrostatic interaction. This is opposite to ssRNA and ssDNA, where a relative contribution of hydrogen bonding between the solid phase and the analyte is increased due to more exposed nucleic bases, thus delaying desorption of ssRNA and ssDNA from the support.

According to the present invention, the nitrogen-containing heterocycle may additionally contain other heteroatoms, such as but not limited to oxygen and/or sulfur. The nitrogen-containing heterocycle is preferably selected from the group consisting of purines, pyrimidines, pyridines, diazoles, triazoles, morpholines and tetrazoles and derivatives thereof. Examples of such compounds include imidazole, benzimidazole, quinoline, isoquinoline and indoline.

In a further preferred embodiment, the nitrogen-containing heterocycle is selected from the group consisting of imidazole, imidazole derivatives, pyridine and pyridine derivatives. Preferred imidazole derivatives include histidine and histamine. A preferred pyridine derivative is 4-mercaptopyridine.

Preferably, the solid phase according to the present invention comprises only one type of multimodal ligand, which means that only identical ligands are comprised in the solid phase. This makes the functionalization of the solid phase easier and allows for a higher resolution when eluting the target single stranded RNA molecule, since the target substance is bound to each multimodal ligand in the same way. Accordingly, the target substance desorbs from all multimodal ligands at similar conditions.

According to the method of the present invention, as a step b), the solid phase is loaded with a sample comprising the target ssRNA. The target ssRNA is the ssRNA intended to be separated from other nucleic acid species by the method of the present invention.

The size and conformation of the target ssRNA is not particularly limited. Typically, the target ssRNA has a size of between 1000 and 25000 bases in length. Similarly, the conformation of the target ssRNa is not particularly limited and the target ssRNA may be present in different conformations, such as linear, circular etc. In preferred embodiments of the present invention, the size and conformation of the target ssRNA is identical throughout one sample. This allows for a clear separation and elution of the target ssRNA in one elution peak.

The sample comprising the target single stranded RNA molecules may, for example, be the result of an in vitro transcription process or the result of an alkaline lysis of a cell suspension. The sample may be used as obtained or may be subjected to a prepurification process to remove proteins and other non-nucleic acid molecules.

In a further preferred embodiment, the target single stranded RNA molecule is an mRNA molecule.

In addition to ssRNA, the sample to be loaded on the solid phase may comprise one or more of single stranded DNA molecules, double stranded DNA molecules, double stranded RNA molecules and plasmids as the other nucleic acid species. The sample may also contain other biological molecules (such as carbohydrates, lipids, proteins, etc.) and/or small organic compounds (such as nucleotides, sugars etc.).

Before loading the sample onto the solid phase, the sample may be equilibrated to a suitable pH value. The pH value to be chosen thereby depends on the solid phase to be used and the kind of multimodal ligand comprised in the solid phase. Preferably, the sample is equilibrated to a pH value 2 units below the pKa of the multimodal ligand comprised in the solid phase.

The kind of buffer used is not particularly limited and conventional buffers used for liquid chromatography can be used as long as they satisfy the above pH conditions. Buffers used can include TRIS, bis-TRIS-propane, 4-(2-hydroxyethyl)-1-piperazineethanesulfonic acid (HEPES), 2-(N-morpholino)ethanesulfonic acid (MES), phosphate, acetate, among others. Furthermore, the equilibration and loading buffers may contain additives as needed and as described herein below.

Additionally, before loading the sample onto the solid phase, the solid phase may be equilibrated to a suitable pH value, preferably 2 units below the pKa value of the multimodal ligand to maximize the charge and capacity of the multimodal ligand. In a preferred embodiment, the solid phase is adjusted to the same pH value as the sample to be loaded onto the solid phase. In this way, acid-base reactions between the sample and the solid phase can be avoided.

In addition or as an alternative, the inclusion of different salts in the equilibration and/or loading buffers is preferred. For example 50 mM sodium phosphate or sodium chloride may be included in the buffers.

After the loading of the sample, unbound species may be washed away by rinsing the solid phase with clean equilibration buffer. This equilibration buffer has preferably the same composition as the buffer that has been used to equilibrate the solid phase and the sample solution.

After the first washing step of rinsing away unbound species, a second washing step may be applied at a higher pH value than the pH value the solid phase is equilibrated to, but below the elution pH of the target ssRNA. This washing step is capable of removing contaminants such as other nucleic acids or proteins, which bind more loosely to the solid phase than the target ssRNA.

In addition or as an alternative, the second washing step may be performed with a buffer having a high salt concentration in order to selectively elute proteins and/or other contaminating species, but not the target ssRNA. For example, washes with a high NaCl concentration (above 0.2 M) can elute some loosely bound contaminants from the surface, for example dsRNA and dsDNA molecules. The concentration of the salt in the wash buffer can have a higher or lower ionic strength compared to the ionic strength of the elution buffer.

In the final step of the method of the present invention, the target single stranded RNA molecule is selectively separated and eluted by applying an ascending pH gradient nearing or crossing the pKa of the multimodal ligand. This ascending pH gradient may be applied in a continuous manner, a stepwise manner, or in a manner comprising continuous and stepwise sections.

The elution buffer may have a similar composition to the buffer used for equilibrating the sample and the solid phase and differs at least in its pH value. It might additionally differ in salt concentration and/or the type of salt contained in the buffer, its conductivity and/or additives added.

As outlined above, when increasing the pH value of the elution buffer nearing or crossing the pKa of the multimodal ligand, the multimodal ligand gradually loses its positive charge, thereby reducing the ion-exchange binding strength towards nucleic acid species. This charge loss, however, does not happen in a concerted fashion for all multimodal ligands at the same time when crossing the pKa value, but the change represents more of a statistical transition of the multimodal ligands from positive charge to neutral around the pKa value of the multimodal ligand. The pH range of charge loss extends from around 2 pH units below the pKa value of the ligand, where 99% of the multimodal ligand molecules are positively charged, to around 2 pH units above the pKa value of the ligand, where 99% of the multimodal ligand molecules are uncharged. This, in combination with the hydrogen bonding and hydrophobic interactions, allows for the separation of the target single stranded RNA molecules from other nucleic acid species having a similarly charged structure and hydrogen bonding potential, such as single stranded DNA.

In view of the above, the starting and end values of the pH gradient are not particularly limited, as long as the pH gradient is ascending, i.e. starts at a lower pH and finishes at a higher pH than the initial pH.

In a preferred embodiment, the pH gradient starts below the pKa value of the multimodal ligand and finishes above the pKa value, where the pH gradient is achieved in approximately 4 pH units. This does, however, not necessarily mean, that the pH gradient crosses the pKa value in the middle.

In an embodiment of the present invention, the separating and eluting of the target single stranded RNA molecules is performed by applying an additional ascending salt gradient in combination with the ascending pH gradient. This variation can further increase the separation efficiency of the method of the present invention because the increasing salt concentration decreases the electrostatic interactions between the multimodal ligands and the nucleic acid species and thus lowers the pH necessary for elution of the target single stranded RNA.

In an embodiment of the present invention, the separating and eluting of the target single stranded RNA molecules is performed by applying an ascending pH gradient while maintaining a salt concentration, which does not elute target ssRNA at the initial pH. For example, a salt concentration of 1M NaCl may be employed. This variation can decrease the elution pH of the target ssRNA molecule, because high salt concentration weakens the electrostatic interaction between biomolecules and the multimodal ligand.

The salt used is not particularly limited and any salt conventionally used for chromatography can be employed, including any ions from the Hofmeister series. Preferably, the salt is a monovalent or divalent salt. The cation of the salt is preferably selected from alkaline metal cations, alkaline earth metal cations, transition metal cations and nitrogen-based cations. The anion of the salt is preferably selected from halogen anions (e.g. chloride, bromide, iodide, etc.) or inorganic acid anions (e.g. phosphate, phosphite, sulfate, sulphite, nitrate, nitrite, chlorate, carbonate, etc.). In specific embodiments, the salt is NaH₂PO₄ or NaCl.

In the method according to the present invention, multivalent cations, such as Mg²⁺, Zn²⁺, Ca²⁺ may be added to the loading and elution buffers in order to increase the resolution between eluted species and to improve the separation selectivity.

Additionally, in the method according to the present invention, metal chelators, such as ethylenediaminetetraacetic acid (EDTA), citric acid, ethylene glycol-bis(2-aminoethylether)-N,N,N',N'-tetraacetic acids (EGTA), tris(2-aminoethyl)amine (TREN) may be added to the loading and elution buffers in order to increase the resolution between eluted species and to change the separation selectivity.

Furthermore, in the method according to the present invention, additives, which alter the interactions between biomolecules themselves and/or between the solid phase and the biomolecules, may be added to the loading and/elution buffers. Examples of such additives include amino acids (e.g. arginine), carbohydrates (e.g. sorbitol), denaturants (e.g. urea, guanidine), organic acids, nitrogen-containing heterocycles (e.g. imidazole) and organic solvents (e.g. isopropanol).

As outlined above, the described method is also capable of separating double stranded RNA molecules by size. Accordingly, the present invention further includes a method of separating double stranded RNA molecules by size, comprising the steps of:
a) providing a solid phase comprising a multimodal ligand having a pKa value of 4.0 to 8.5, the multimodal ligand comprising a nitrogen-containing heterocycle;
b) loading a sample to the solid phase, the sample containing two or more double stranded RNA molecules of different length; and
c) separating and eluting the double stranded RNA molecules by size by applying an ascending pH gradient nearing or crossing the pKa value of the multimodal ligand.

Further modifications of this method include the same as those described for the method of separating single stranded RNA molecules from other nucleic acid species described above.

The present invention including preferred embodiments will now be described in more detail along with the accompanying figures.

### Brief description of the figures

- Figure 1: depicts separation of dsRNA and plasmid dsDNA from mRNA with a histamine-modified monolith by means of an ascending pH gradient at ambient temperature (Example 2).
- Figure 2: depicts size separation of dsRNA and NTPs with a histamine-modified monolith by means of an ascending pH gradient at ambient temperature (Example 3).
- Figure 3: depicts separation of plasmid dsDNA from mRNA with a 4-mercaptopyridine monolith by means of an ascending pH gradient at ambient temperature (Example 5).

In summary, the method of the present invention may be employed for the purpose of performing analytical or preparative applications. The specific chromatography conditions and degree of separation between different DNA/RNA species may differ among conformations and lengths. This can be exploited for more efficient separation with the goal to reach higher resolution (analytics) and/or higher purity of biological molecule (preparative scale).

The present invention will be further illustrated in the following examples without being limited thereto.

### Examples

### Example 1: Preparation of histamine-modified monolith

A histamine-modified monolith was prepared using the following exemplary procedure. Briefly, histamine was immobilized on a monolith via carboxyimidazole (CDI) activation chemistry using a 0.1 mL CIMmic-CDI monolith (BIA Separations d.o.o., catalog number 103.8000-2). The immobilization procedure consisted of pumping the histamine solution through the monolithic columns (10 g histamine dihydrochloride dissolved in 100 ml of 1.0 M NaOH) followed by 48 h thermostating of the column at 25 °C. After the modification the monolith surface was exposed to 0.5 M NaOH to deactivate the residual CDI groups.

### Example 2: Separation of dsRNA and plasmid dsDNA from mRNA with a histamine-modified monolith by means of an ascending pH gradient at ambient temperature

A histamine-modified solid phase in the form of a 0.1 mL monolithic chromatography device was used in combination with a PATfix HPLC system (BIA Separations d.o.o).

The system was operated at ambient temperature. The histamine monolith was equilibrated with 10 mM Tris, 10 mM BTP, pH 6.0. Samples containing dsRNA ladder molecules ranging from 21 bp to 500 bp, plasmid dsDNA of 7300 bp and 1000 bp mRNA ssRNA were prepared by dilution in 10 mM Tris, 10 mM BTP, pH 6.0 and applied to the histamine-modified monolith. A 5 CV wash with 10 mM Tris, 10 mM BTP, pH 6.0 was then applied to displace unbound materials from the channels within the monolith. The monolith was then eluted with a linear gradient to 10 mM Tris, 10 mM BTP, pH 9.5 in 100 CVs at a flowrate of 10 CVs/min at ambient temperature.

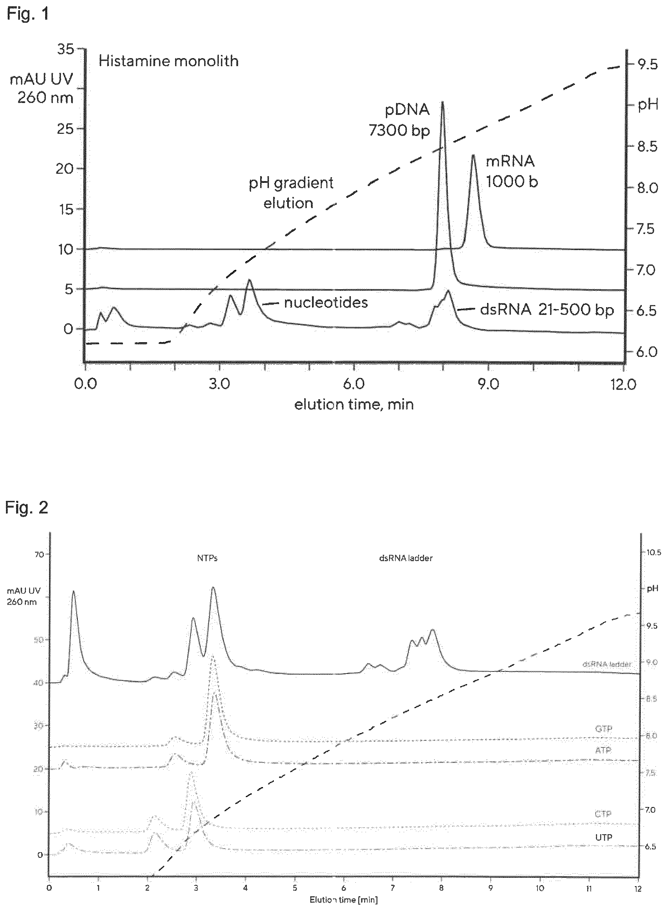

As shown in Figure 1, all the dsRNA molecules and plasmid dsDNA eluted at a pH, where mRNA ssRNA remained bound. mRNA ssRNA eluted at a higher pH value than dsRNA molecules and plasmid dsDNA. Some size separation is observed among dsRNA molecules with additional separation of nucleotides present in the sample, which eluted at a lower pH as other nucleic acid molecules, due to much weaker interactions compared to nucleic acid polymers. The elution recoveries for all nucleic acids used in the separation, were higher than 85 %.

### Example 3: Size separation of dsRNA and NTPs with a histamine-modified monolith by means of an ascending pH gradient at ambient temperature

A histamine-modified solid phase in the form of a 0.1 mL monolithic chromatography device was used in combination with a PATfix HPLC system (BIA Separations d.o.o). The system was operated at ambient temperature. The histamine monolith was equilibrated with 10 mM Tris, 10 mM BTP, 20 mM guanidinium chloride, pH 6.0. Samples containing dsRNA ladder molecules ranging from 21 bp to 500 bp (NEB, N0363S) or individual nucleotide triphosphates (NTPs) adenosine triphosphate (ATP), guanosine triphosphate (GTP), cytidine triphosphate (CTP) and uridine triphosphate (UTP) were prepared by dilution in 10 mM Tris, 10 mM BTP, 20 mM guanidinium chloride, pH 6.0 and applied to the histamine-modified monolith. A wash with 10 mM Tris, 10 mM BTP, 20 mM guanidinium chloride, pH 6.0 was then applied to displace unbound materials from the channels within the monolith. The monolith was then eluted with a linear gradient to 10 mM Tris, 10 mM BTP, 20 mM guanidinium chloride, pH 9.5 in 100 CVs at a flowrate of 10 CVs/min at ambient temperature.

Chaotropic guanidinium was included in the whole separation procedure to improve the size separation of dsRNA molecules, as depicted in Figure 2. Purine NTPs clearly separate from pyrimidine NTPs, with the former eluting at a higher pH value. Additionally, NTPs separate from trace nucleotide diphosphates (NDPs), which elute at a lower pH value than NTPs.

In view of the above, the method of the present invention can be applied to monitoring ssRNA production by means of an IVT reaction as it separates the final product ssRNA, from the template molecule dsDNA and the nucleotides used as reaction substrate.

### Example 4: Preparation of 4-mercaptopyridine-modified monolith

4-mercaptopyridine-modified monolith was prepared by covalent coupling of 4-mercaptopyridine to an epoxide-bearing CIM monolith (BIA Separations d.o.o.). Briefly, 4-mercaptopyridine was immobilized on the monolith via epoxy activation chemistry using a 0.1 mL CIMmic epoxy monolith (BIA Separations d.o.o.). The immobilization procedure consisted of pumping the 4-mercaptopyridine solution through the monolithic column (10% (v/v) 4-mercaptopyridine solution in 96% EtOH) followed by 48 h thermostating of the column at 50 °C. After the modification the monolith surface was exposed to 0.5 M H₂SO₄ for 5 h at 65 °C to deactivate the residual epoxy groups.

### Example 5: Separation of plasmid dsDNA from mRNA with a 4-mercaptopyridine monolith by means of an ascending pH gradient at ambient temperature

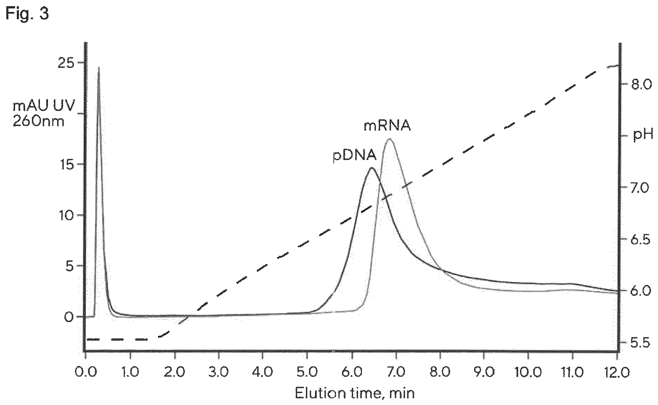

A 4-mercaptopyridine-modified solid phase in the form of a 0.1 mL monolithic chromatography device was used in combination with a PATfix HPLC system (BIA Separations d.o.o). The system was operated at ambient temperature, results are shown in Figure 3. The 4-mercaptopyridine monolith was equilibrated with 10 mM MES, 10 mM HEPES, 7 % (v/v) isopropanol, pH 5.5. Samples containing plasmid dsDNA and mRNA were prepared by dilution in 10 mM MES, 10 mM HEPES, 7 % (v/v) isopropanol, pH 5.5 and applied to the 4-mercaptopyridine-modified monolith. A wash with 10 mM MES, 10 mM HEPES, 7 % (v/v) isopropanol, pH 5.5 was then applied to displace unbound materials from the channels within the monolith. The monolith was then eluted with a linear gradient to 10 mM MES, 10 mM HEPES, 7 % (v/v) isopropanol, pH 8.0 in 100 CVs at a flowrate of 10 CVs/min at ambient temperature.

Isopropanol was included in the separation procedure to improve the elution shape of nucleic acids due to relatively high hydrophobicity of the ligand. This example demonstrates that the method of the present invention is also applicable, but not limited, to pyridine-based multimodal ligands. Additionally, this example demonstrates that the separation conditions, including buffer composition and pH, can be tailored to suit the properties of different ligands.

## Claims

1. A method of separating single stranded RNA molecules from other nucleic acid species, comprising the steps of:
a) providing a solid phase comprising a multimodal ligand having a pKa value of 4.0 to 8.5, the multimodal ligand comprising a nitrogen-containing heterocycle;
b) loading a sample to the solid phase, the sample containing the target single stranded RNA molecules to be separated; and
c) separating and eluting the target single stranded RNA molecules by applying an ascending pH gradient nearing or crossing the pKa value of the multimodal ligand.

2. The method according to claim 1, wherein the multimodal ligand has a pKa value of 5.0 to 8.0.

3. The method according to claim 1 or 2, wherein the nitrogen-containing heterocycle is an aromatic nitrogen-containing heterocycle.

4. The method according to any one of claims 1 to 3, wherein the nitrogen-containing heterocycle is selected from the group consisting of purines, pyrimidines, pyridines, diazoles, triazoles, morpholines, and tetrazoles.

5. The method according to claim 4, wherein the nitrogen-containing heterocycle is selected from the group consisting of imidazole, imidazole derivatives, pyridine or pyridine derivatives.

6. The method according to any one of claims 1 to 5, wherein the solid phase is a chromatographic column comprising a solid stationary phase.

7. The method according to any one of claims 1 to 6, wherein the sample is loaded on the solid phase at a pH lower than the pKa of the multimodal ligand.

8. The method according to any one of claims 1 to 7, wherein the target single stranded RNA molecule is an mRNA molecule or a derivative of single stranded RNA or mRNA molecule.

9. The method according to any one of claims 1 to 8, wherein the sample to be loaded on the solid phase comprises one or more of single stranded DNA molecules, double stranded DNA molecules, double stranded RNA molecules and plasmids as the other nucleic acid species.

10. The method according to any one of claims 1 to 9, further comprising after step b) and before step c) a step b1) of washing off unbound species.

11. The method according to claim 10, further comprising after step b1) and before step c) a step b2) of washing with a solution having a higher pH value than the solution used for loading the sample to the solid phase in step b) but lower than the elution pH of the target single stranded RNA molecules.

12. The method according to any one of claims 1 to 11, wherein the ascending pH gradient starts at a pH value at least one pH unit below the pKa value of the multimodal ligand and finishes at a pH value at least one pH unit above the pKa value of the multimodal ligand.

13. The method according to any one of claims 1 to 12, wherein the separating and eluting of the target single stranded RNA molecules is performed by applying an ascending salt gradient in combination with the ascending pH gradient.

14. Use of a solid phase comprising a multimodal ligand having a pKa value of 4.0 to 8.5, the multimodal ligand comprising a nitrogen-containing heterocycle, for separating single stranded RNA molecules from other nucleic acid species by liquid chromatography.

15. The use according to claim 14, wherein the multimodal ligand is imidazole or an imidazole derivative.

16. A method of separating double stranded RNA molecules by size, comprising the steps of:
a) providing a solid phase comprising a multimodal ligand having a pKa value of 4.0 to 8.5, the multimodal ligand comprising a nitrogen-containing heterocycle;
b) loading a sample to the solid phase, the sample containing two or more double stranded RNA molecules of different length; and
c) separating and eluting the double stranded RNA molecules by size by applying an ascending pH gradient nearing or crossing the pKa value of the multimodal ligand.

## Patentansprüche

1. Verfahren zur Trennung von einzelsträngigen RNA-Molekülen von anderen Nukleinsäurespezies, das die folgenden Schritte umfasst:
a) Bereitstellen einer Festphase, die einen multimodalen Liganden umfasst, der einen pKa-Wert von 4,0 bis 8,5 aufweist, wobei der multimodale Ligand einen stickstoffhaltigen Heterocyclus umfasst;
b) Laden einer Probe in die Festphase, wobei die Probe die zu trennenden einzelsträngigen Ziel-RNA-Moleküle enthält; und
c) Trennen und Eluieren der einzelsträngigen Ziel-RNA-Moleküle durch Anwenden eines aufsteigenden pH-Wert-Gradienten, der sich an den pKa-Wert des multimodalen Liganden annähert oder diesen überschreitet.

2. Verfahren nach Anspruch 1, wobei der multimodale Ligand einen pKa-Wert von 5,0 bis 8,0 aufweist.

3. Verfahren nach Anspruch 1 oder 2, wobei der stickstoffhaltige Heterocyclus ein aromatischer stickstoffhaltiger Heterocyclus ist.

4. Verfahren nach einem der Ansprüche 1 bis 3, wobei der stickstoffhaltige Heterocyclus ausgewählt wird aus der Gruppe, die besteht aus Purinen, Pyrimidinen, Pyridinen, Diazolen, Triazolen, Morpholinen und Tetrazolen.

5. Verfahren nach Anspruch 4, wobei der stickstoffhaltige Heterocyclus ausgewählt wird aus der Gruppe, die besteht aus Imidazol, Imidazol-Derivaten, Pyridin oder Pyridin-Derivaten.

6. Verfahren nach einem der Ansprüche 1 bis 5, wobei die Festphase eine chromatographische Säule ist, die eine stationäre Festphase umfasst.

7. Verfahren nach einem der Ansprüche 1 bis 6, wobei die Probe auf die Festphase bei einem pH-Wert geladen wird, der niedriger als der pKa-Wert des multimodalen Liganden ist.

8. Verfahren nach einem der Ansprüche 1 bis 7, wobei das einzelsträngige Ziel-RNA-Molekül ein mRNA-Molekül oder ein Derivat von einem einzelsträngigen RNA- oder mRNA-Molekül ist.

9. Verfahren nach einem der Ansprüche 1 bis 8, wobei die auf die Festphase zu ladende Probe eines oder mehrere von einzelsträngigen DNA-Molekülen, doppelsträngigen DNA-Molekülen, doppelsträngigen RNA-Molekülen und Plasmiden als die anderen Nukleinsäurespezies umfasst.

10. Verfahren nach einem der Ansprüche 1 bis 9, ferner umfassend, nach dem Schritt b) und vor dem Schritt c), einen Schritt b1) zum Abwaschen ungebundener Spezies.

11. Verfahren nach Anspruch 10, ferner umfassend, nach dem Schritt b1) und vor dem Schritt c), einen Schritt b2) zum Waschen mit einer Lösung, die einen höheren pH-Wert aufweist als die Lösung, die zum Laden der Probe in die Festphase in Schritt b) verwendet wird, aber niedriger als der Elutions-pH-Wert der einzelsträngigen Ziel-RNA-Moleküle ist.

12. Verfahren nach einem der Ansprüche 1 bis 11, wobei der aufsteigende pH-Wert-Gradient bei einem pH-Wert beginnt, der mindestens eine pH-Wert-Einheit unter dem pKa-Wert des multimodalen Liganden liegt, und bei einem pH-Wert endet, der mindestens eine pH-Wert-Einheit über dem pKa-Wert des multimodalen Liganden endet.

13. Verfahren nach einem der Ansprüche 1 bis 12, wobei das Trennen und Eluieren der einzelsträngigen Ziel-RNA-Moleküle durch Anwenden eines aufsteigenden Salzgradienten in Kombination mit dem aufsteigenden pH-Wert-Gradienten durchgeführt wird.

14. Verwendung einer Festphase, die einen multimodalen Liganden umfasst, der einen pKa-Wert von 4,0 bis 8,5 aufweist, wobei der multimodale Ligand einen stickstoffhaltigen Heterocyclus umfasst, zum Trennen einzelsträngiger RNA-Moleküle von anderen Nukleinsäurespezies durch Flüssigchromatographie.

15. Verwendung nach Anspruch 14, wobei der multimodale Ligand Imidazol oder ein Imidazol-Derivat ist.

16. Verfahren zum Trennen doppelsträngiger RNA-Moleküle nach Größe, das die folgenden Schritte umfasst:
a) Bereitstellen einer Festphase, die einen multimodalen Liganden umfasst, der einen pKa-Wert von 4,0 bis 8,5 aufweist, wobei der multimodale Ligand einen stickstoffhaltigen Heterocyclus umfasst;
b) Laden einer Probe in die Festphase, wobei die Probe zwei oder mehr doppelsträngige RNA-Moleküle mit unterschiedlicher Länge enthält; und
c) Trennen und Eluieren der doppelsträngigen RNA-Moleküle nach Größe durch Anwenden eines aufsteigenden pH-Wert-Gradienten, der sich an den pKa-Wert des multimodalen Liganden annähert oder diesen überschreitet.

## Revendications

1. Procédé de séparation de molécules d'ARN simple brin d'avec d'autres espèces d'acides nucléiques, comprenant les étapes de :
a) fourniture d'une phase solide comprenant un ligand multimodal ayant une valeur de pKa de 4,0 à 8,5, le ligand multimodal comprenant un hétérocycle azoté ;
b) chargement d'un échantillon sur la phase solide, l'échantillon comprenant les molécules d'ARN simple brin cibles devant être séparées ; et
c) séparation et élution des molécules d'ARN simple brin cibles par application d'un gradient de pH ascendant se rapprochant ou croisant la valeur de pKa du ligand multimodal.

2. Procédé selon la revendication 1, dans lequel le ligand multimodal a une valeur de pKa de 5,0 à 8,0.

3. Procédé selon la revendication 1 ou 2, dans lequel l'hétérocycle azoté est un hétérocycle azoté aromatique.

4. Procédé selon l'une quelconque des revendications 1 à 3, dans lequel l'hétérocycle azoté est choisi dans le groupe constitué par les purines, les pyrimidines, les pyridines, les diazoles, les triazoles, les morpholines, et les tétrazoles.

5. Procédé selon la revendication 4, dans lequel l'hétérocycle azoté est choisi dans le groupe constitué par l'imidazole, les dérivés d'imidazole, la pyridine et les dérivés de pyridine.

6. Procédé selon l'une quelconque des revendications 1 à 5, dans lequel la phase solide est une colonne chromatographique comprenant une phase stationnaire solide.

7. Procédé selon l'une quelconque des revendications 1 à 6, dans lequel l'échantillon est chargé sur la phase solide à un pH inférieur au pKa du ligand multimodal.

8. Procédé selon l'une quelconque des revendications 1 à 7, dans lequel la molécule d'ARN simple brin cible est une molécule d'ARNm ou un dérivé de molécule d'ARNm ou d'ARN simple brin.

9. Procédé selon l'une quelconque des revendications 1 à 8, dans lequel l'échantillon devant être chargé sur la phase solide comprend un ou plusieurs parmi des molécules d'ADN simple brin, des molécules d'ADN double brin, des molécules d'ARN double brin et des plasmides en tant qu'autres espèces d'acides nucléiques.

10. Procédé selon l'une quelconque des revendications 1 à 9, comprenant en outre, après l'étape b) et avant l'étape c), une étape b1) d'élimination par lavage des espèces non liées.

11. Procédé selon la revendication 10, comprenant en outre, après l'étape b1) et avant l'étape c), une étape b2) de lavage avec une solution ayant une valeur de pH supérieure à celle de la solution utilisée pour le chargement de l'échantillon sur la phase solide dans l'étape b) mais inférieure au pH d'élution des molécules d'ARN simple brin cibles.

12. Procédé selon l'une quelconque des revendications 1 à 11, dans lequel le gradient de pH ascendant commence à une valeur de pH inférieure d'au moins une unité de pH à la valeur de pKa du ligand multimodal et se finit à une valeur de pH supérieure d'au moins une unité de pH à la valeur de pKa du ligand multimodal.

13. Procédé selon l'une quelconque des revendications 1 à 12, dans lequel la séparation et l'élution des molécules d'ARN simple brin cibles sont effectuées par application d'un gradient de sel ascendant en combinaison avec le gradient de pH ascendant.

14. Utilisation d'une phase solide comprenant un ligand multimodal ayant une valeur de pKa de 4,0 à 8,5, le ligand multimodal comprenant un hétérocycle azoté, pour séparer des molécules d'ARN simple brin d'avec d'autres espèces d'acides nucléiques par chromatographie liquide.

15. Utilisation selon la revendication 14, dans laquelle le ligand multimodal est l'imidazole ou un dérivé d'imidazole.

16. Procédé pour séparer des molécules d'ARN double brin en fonction de leur taille, comprenant les étapes de :
a) fourniture d'une phase solide comprenant un ligand multimodal ayant une valeur de pKa de 4,0 à 8,5, le ligand multimodal comprenant un hétérocycle azoté ;
b) chargement d'un échantillon sur la phase solide, l'échantillon comprenant deux ou plus de deux molécules d'ARN double brin ayant des longueurs différentes ; et
c) séparation et élution des molécules d'ARN double brin en fonction de leur taille par application d'un gradient de pH ascendant se rapprochant ou croisant la valeur de pKa du ligand multimodal.
